# EUROPEAN PATENT SPECIFICATION

(11) **EP 2 887 242 B1**
(45) Date of publication and mention of the grant of the patent: **07.11.2018**
(21) Application number: 13199350.3
(22) Date of filing: 23.12.2013
(51) Int. Cl.: G08B 29/16

(54) **Hand call unit**
Handanrufeinheit
Unité d'appel à la main

(43) Date of publication of application: 24.06.2015
(73) Proprietor: Televic Healthcare NV, 8870 Izegem (BE)
(72) Inventor: Verhaeghe, Geert, 8680 Koekelare (BE); Vanoosthuyse, Alexander, 8500 Kortrijk (BE)
(74) Representative: Van Bladel, Marc

(56) References cited:
- EP-A2- 1 400 941
- GB-A- 2 251 106
- US-A1- 2002 044 043
- US-B1- 6 304 797

## Description

### Field of the invention

The present invention is generally related to the field of hand call units that are part of a nurse call system and that should be capable of communicating with other parts of the nurse call system.

### Background of the invention

As nurse call systems should allow people to quickly call assistance in emergency situations, security against system failure and the ability to operate flawlessly at any time are of utmost importance. The VDE standard (DIN VDE 0834) takes these considerations into account. It prescribes that the call system be completely independent of parts not being part of the system. Data transfer with other systems is only allowed when using the own interfaces of the system.

Especially for the nurse call environment VDE regulation stipulates the need for reliable operation. In the past VDE regulations were merely met by implementing wire monitoring schemes, detecting the breaking of wires and mechanical switches within the nurse call system.

Due to the above-mentioned VDE regulations third-party (network) components cannot be part of the core of the nurse call system. In prior art solutions therefore proprietary network switches have been implemented or network switching technology was added in the nurse call devices.

Japanese application JP2009135680 is concerned with ensuring stable radio communication between devices of a nurse call system by enhancing reliability of data to be transmitted and received. However, in the proposed solution only one communication path is available.

JP2013012808 relates to a call controller for a nurse call system. A solution is proposed that allows making a call to an Internet Protocol (IP) telephone that has failed to receive an INVITE command transmitted first from a Session Initiation Protocol (SIP) server.

Application US2009/217080 discloses a distributed fault tolerant architecture for a healthcare communication system. The healthcare communication system includes a first plurality of computer devices, such as patient stations, staff stations, and a master station, that are operable as a nurse call system. The first plurality of computer devices may have core nurse call functionality residing on an embedded computing platform. A second plurality of computer devices provides the first plurality of computer devices with additional functionality via software plug-ins that are transmitted to the first plurality of computer devices. A fall-back system for a full-IP system is so proposed.

US6304797 relates to an automated medication dispenser with a remote patient monitoring system. A dispensing unit is disclosed comprising a carousel defining a plurality of compartments each adapted to store a dose of medication. Also disclosed is an emergency pendant to be carried by a patient and coupled to a security system. By pressing a button the patient can launch an alarm signal.

Application EP1400941 discloses a nurse call system that meets the VDE safety requirements and is cost efficient. The system utilizes a digital telecommunications network coupled between terminal units incorporating call initiation devices and an application server and connected to a function monitoring device, providing periodic test data transmitted to the application server, for activation of a fault indicator upon incorrect handling of the test data.

In US2002/044043 a patient care and communication system is shown including a central station provided with means for facilitating visual and data communication and in connection with a number of remote stations. In event of a failure the stations can operate in a local mode using a fail safe bus. In this bus several options are available for establishing connection.

In document GB2251106 is presented a centralized monitoring unit for a multi-point detection system, e.g. nurse call or intruder detection system. It comprises a data input/output unit for connection to a plurality of detectors, such as a nurse call station, in daisy-chained manner, to permit an addressing unit to supply a respective interrogation signal to each detector in turn, responses causing code numbers indicative of actuated detectors to be displayed.

There is a need for providing fall-back measures when the preferred path of operation cannot be guaranteed.

### Summary of the invention

It is an object of embodiments of the present invention to provide for a nurse call system with a handset that allows for communication with other parts of a nurse call system in a more reliable way.

The above objective is accomplished by the solution according to the present invention.

In a first aspect the invention relates to a nurse call system as in claim 1.

The proposed solution indeed offers a redundant path. A first path is provided by a passive electrical connection between the handset and the central controller through the at least one input/output device of the nurse call system. By monitoring the status indicator the handset can detect an error or anomaly in the functioning of the nurse call system. In case a faulty operation is observed, the processor in the handset can switch to a second communication path using a network connection between the handset and the central controller.

In a preferred embodiment the passive electrical connection complies with the DIN VDE 0834 standard.

In an advantageous embodiment the hand call unit comprises a display. Preferably the display is arranged for displaying an indication of the faulty operation.

In a preferred embodiment the processing means is adapted for detecting a network fault in the second communication link.

In one embodiment the status indicator is arranged for emitting a dedicated pattern of light to signal the faulty operation.

In one embodiment the second communication link is an Ethernet connection.

Advantageously, the call button is a normally closed button. The processing means is in that case preferably arranged for detecting false operation of the call button.

For purposes of summarizing the invention and the advantages achieved over the prior art, certain objects and advantages of the invention have been described herein above. Of course, it is to be understood that not necessarily all such objects or advantages may be achieved in accordance with any particular embodiment of the invention. Thus, for example, those skilled in the art will recognize that the invention may be embodied or carried out in a manner that achieves or optimizes one advantage or group of advantages as taught herein without necessarily achieving other objects or advantages as may be taught or suggested herein.

The above and other aspects of the invention will be apparent from and elucidated with reference to the embodiment(s) described hereinafter.

### Brief description of the drawings

The invention will now be described further, by way of example, with reference to the accompanying drawings, wherein like reference numerals refer to like elements in the various figures.
Fig.1 illustrates a general block scheme of a nurse call system and its main components.
Fig.2 illustrates an embodiment of the hand call unit according to the invention.
Fig.3 illustrates an embodiment of the hand call unit connected to other components of a nurse call system.
Fig.4 illustrates a flowchart of the reliable sending of a nurse call alarm.

### Detailed description of illustrative embodiments

The present invention will be described with respect to particular embodiments and with reference to certain drawings but the invention is not limited thereto but only by the claims.

Furthermore, the terms first, second and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

It should be noted that the use of particular terminology when describing certain features or aspects of the invention should not be taken to imply that the terminology is being redefined herein to be restricted to include any specific characteristics of the features or aspects of the invention with which that terminology is associated.

In the description provided herein, numerous specific details are set forth. However, it is understood that embodiments of the invention may be practiced without these specific details. In other instances, well-known methods, structures and techniques have not been shown in detail in order not to obscure an understanding of this description.

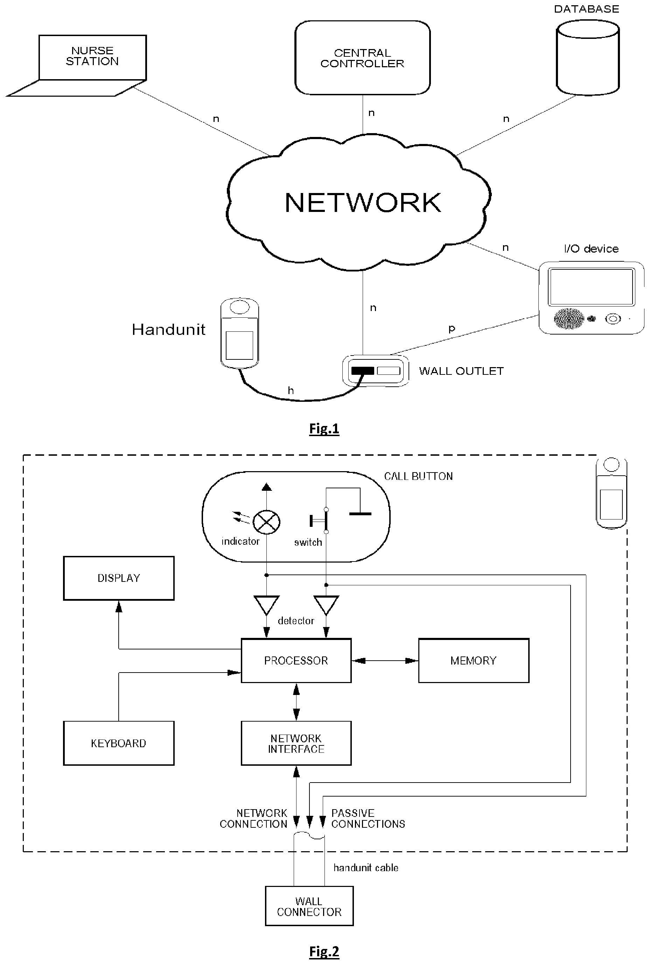

The present invention relates to a hand call unit for use in a nurse call system. The nurse call system as considered in this invention can be expanded to cater different needs such as intercom, domotics control, access control and more. A block diagram of a nurse call system enhanced with such functions is shown in Fig.1.

Fig.1 illustrates a nurse call system with its main components. The hand call unit is connected to a wall outlet via a cable h, which can be seen as a hybrid cable comprising both a passive connection p and a network connection n. In the topology of Fig.1 the outlet is in connection with the I/O device via a passive link p and in connection with the other components of the nurse call system through the network connection n. The other components of the nurse call system are shown in the figure : nurse station, central controller and a database. These components are also linked with each other through network connections n. In the database it is possible to store information concerning errors detected in the nurse call system, which can be useful for later analysis. Immediate actions to be taken are typically displayed at the nurse station. These messages may as well be forwarded by the central controller to third party systems like a GSM network, DECT, pagers etc.

The nurse call messages (commonly referred to as alarms) are sent to the central controller for processing and distributing. The central controller is also able to distribute the error messages alongside with the other nurse call message to other input/output devices in the system if instructed to do so. A vital part in a nurse call system is formed by the devices the patients have access to, i.e. the hand call unit.

Due to their known non-deterministic nature Ethernet networks have not been used for implementing nurse call systems for a long time. In these systems the Ethernet was at most used alongside of the nurse call system in order to bring added value (like entertainment, VoIP telephony etc.) to the patient environment.

The saving potential of using a common network infrastructure like Ethernet for everything in the hospital is however quite obvious : standard components and lines are shared, value added services and convenient functions can be individually brought to the point of care. Standard equipment simplifies service and allows easily exchanging or expanding devices in rooms. Additional nurse call functions as presence or more common functions as VoIP-telephony can then readily be made available.

The present invention presents a step in that direction. It proposes a second connection between the handset and the I/O device. Apart from the passive VDE (DIN VDE 0834) compliant connection, there is also a connection via a nurse call network, for example via an Ethernet connection.

An embodiment of the hand call unit according to the invention is schematically represented in Fig.2. It comprises a push button, a keypad and a display. A wired connection is provided between the hand call unit and an I/O device in the room of the patient. The wiring is compliant with the VDE standard.

In the previous explanation of the working principles, the first path was always referred to as a passive connection. It may be clearly understood that "passive" in this context means a link that conveys a button and indicator status to an input/output device in a reliable way. In the simplest way this can be achieved with a single physical electrical connection like a copper wire. In a more elaborate way it can be achieved by implementing a virtual connection through a proprietary secured software link between the handset and the input/output device, making abstraction of the physical medium which is used for conveying the status of the button and indicator over the link.

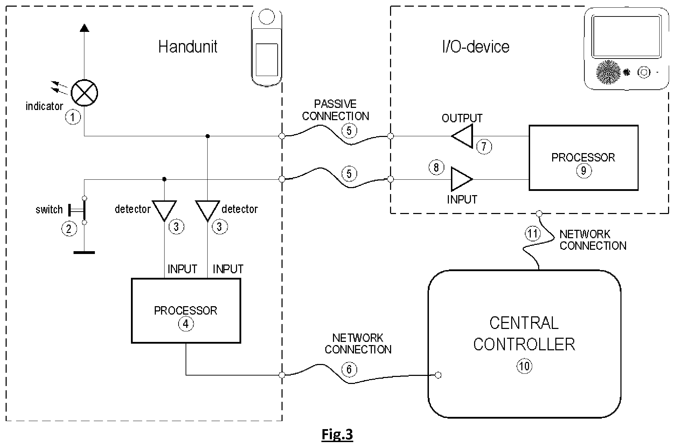

Fig.3 schematically shows the connection of the handset to the rest of the nurse call system. When a patient needs assistance, he pushes the button (2) on the hand unit. When the call is accepted in the system, the indicator (1) in the hand unit will blink on a predefined rhythm. The indicator (1) is typically a LED behind or close to the button. Its primary function is to give feedback to the user that his call has been accepted. The button (2) is typically a normally closed (NC) button so that cabling and button failures can be accounted for. Detectors (3) are arranged to detect changes of the buttons and of the LED indicator. The hand call unit is further provided with a processor (4), which allows for (possibly proprietary) software to run on the hand unit to monitor the indicator (1) and the call button (2).

The hand call unit has a passive connection with the input/output (I/O) device. The passive connection is VDE compliant. In Fig.3 the I/O device is shown as a part of a terminal. In another possible implementation it is a stand-alone device. The above-mentioned terminal is a representation of several possible implementations of these nurse call terminals which merely depend on the hardware and/or software options installed. These options can be the availability of VoIP, touchscreen, etc.

In the solution according to the invention a second communication link is provided between the hand call unit and the central controller of the nurse call system (10). A network connection (6) is created, e.g. by Ethernet cabling, between the hand unit and the nurse call system. Between the I/O device and the central controller a network connection (11) is available.

The I/O device comprises an output (7), which is a driver that puts current on the wiring. The input (8) of the I/O device is adapted for detecting incoming signals.

As passive, e.g. wired, connections is still a more robust way of handling mission critical systems, the primary communication mechanism in the hand call unit of this invention is still electrical wiring. The invention proposes the use of a network connection (e.g. an Ethernet connection) as fall-back mechanism in this case.

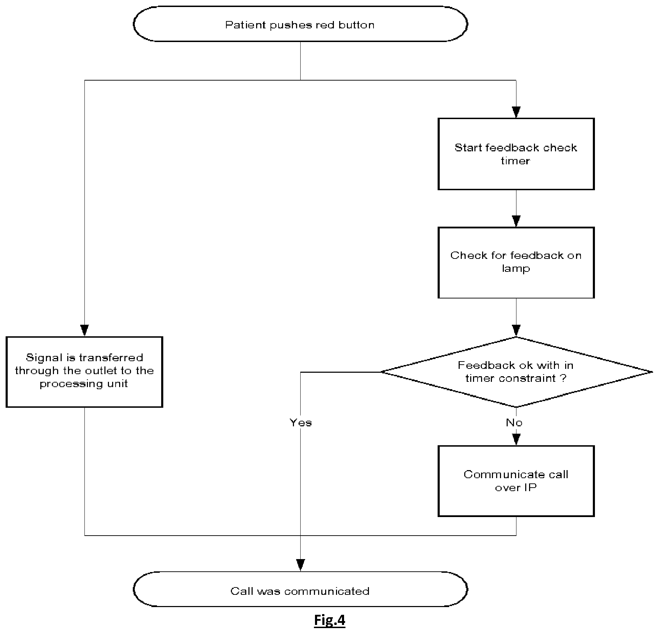

Fig.4 shows a flowchart for reliably sending a nurse call alarm. As a first means of verification the feedback after pressing the button is monitored using the detector on the wiring to the LED. This is done by first detecting the input change. Afterwards a timer is started on the processor of the hand set. This timer waits for change of the detector on the LED side. If the LED changes within the given time, the processor can reliably conclude that the nurse call alarm was successfully processed by the other I/O device. If the LED does not change state (i.e. LED does not blink), the processor picks this up and start a network communication link session, e.g. over IP or over Ethernet. The precondition for this fall-back mechanism is that the network connection is still present and functioning.

If a fault is detected in both communication links within a fixed time period (e.g. within an interval of 3 seconds), the hand call unit can safely assume it is in an isolated mode. In an isolated mode both communication paths are not available. If the handset is in an isolated mode, it will not start to blink the LED in the usual way, however it will do something else, e.g. emit a predefined light pattern. By default, the buzzer sound may be generated. As a secondary action, the entire workflow is restarted after 10 seconds, until a nurse removes the alarm by accessing the nurse call menus on the handset.

In the description above the second communication link over a network has been represented by an Ethernet connection. However, the skilled person will readily appreciate this is merely an example. Other options for implementing the second communication link may be any wired or wireless network connection, like a local area network, a WiFi network, a proprietary network etc...

The proposed solution takes the reliability to a higher level by using the network capabilities of the system as backup for the traditional wire monitoring scheme.

The above-mentioned fall-back mechanism enables the system to detect and account for various failures which may occur in a nurse call system. Concerning the call button, the most commonly failure is wear-out of the contact mechanism by aging or overstressing the button. When this happens the system detects that the button is no longer connect to ground over a longer period in time and signals this as a faulty status. The same mechanism accounts also for the detection of any wire break between the call-button and the input/output device.

The combination of the first passive link together with the network link also enables the nurse call system to detect if the absence of the call button presence detection is due to removal of the handset or due to the failure of a part of the system.

Using the fall-back mechanism described above, not only the button status is monitored, but also the status of the indicator. As the typical failure mode of an indicator (may it be a LED or light bulb) is that it becomes an open circuit, which can be detected in the provided hardware.

By using the above additional detection mechanisms, the system can even detect and signal fault conditions before the user actually presses the button, leading towards a more reliable nurse call communication system.

As stated above the failures can be signalled to the system by using the second communication path. In addition to this, appropriate messages can be generated on the handset to draw the user's attention, being the patient and/or the nurse, to inform him of a fault condition detected in the system, even if the handset is not in isolated mode. This message can be something displayed on the LCD and/or a sound or message played through the handset speaker.

When cabling breaks or is faulty, the system detects this on the hardware level. When detecting a problem with the cabling to the processing unit, the handset automatically falls back to sending a nurse call message over the nurse call network. This way, all parties listening to the network message know a call is present on the handset. Using an optional retransmission mechanism after a given period of time, all parties are informed in a repeated way of the state of the nurse call message on the handset, until the fault is removed, thus increasing the reliability of the nurse call message on the system level.

Due to the way the hardware has been built, by monitoring inputs and outputs, the hand call unit of the invention manages to provide a fall-back mechanism on the most mission critical part of the system, namely the nurse call button. The preferred way of communicating a nurse call, has thereby not been altered : it is still the wired way of working. As an added value, the possibility is created for the handset to detect the feedback given by the processing unit (in the I/O device) and evaluate.

While the invention has been illustrated and described in detail in the drawings and foregoing description, such illustration and description are to be considered illustrative or exemplary and not restrictive. The foregoing description details certain embodiments of the invention. It will be appreciated, however, that no matter how detailed the foregoing appears in text, the invention may be practiced in many ways. The invention is not limited to the disclosed embodiments.

Other variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. Nurse call system comprising
- a central controller (10) arranged for processing and distributing nurse call messages,
- an input/output device connected with said central controller via a first communication link (11) of a nurse call network,
- a handset for making a nurse call, said handset being arranged for communicating with said input/output device via a wired electrical connection (5) and comprising a call button (2) provided with a status indicator (1) for indicating a call being present or not, and a processing means (4) capable of monitoring said status indicator and said call button, said handset being connected to said central controller via said first communication link through said input/output device,
**characterised in that** said processing means of said handset is arranged for, on detection at hardware level of a faulty operation of the communication between the handset and the input/output device, establishing as a second path a second network communication link (6) between said handset and said central controller (10), whereby said input/output device is avoided.

2. Nurse call system as in claim 1, wherein said wired electrical connection is compliant with the DIN VDE 0834 standard.

3. Nurse call system as in claim 1 or 2, wherein said handset comprises a display.

4. Nurse call system as in claim 3, wherein said display is arranged for displaying an indication of said faulty operation.

5. Nurse call system as in any of the previous claims, wherein said processing means (4) is adapted for detecting a network fault in said second communication link.

6. Nurse call system as in claim 5, wherein said handset is arranged for providing feedback on detection of said network fault.

7. Nurse call system as in any of the previous claims, wherein said status indicator is arranged for emitting a dedicated pattern of light to signal said faulty operation.

8. Nurse call system as in any of the previous claims, wherein said second communication link is an Ethernet connection.

9. Nurse call system as in any of the previous claims, wherein said call button (2) is a normally closed button.

10. Nurse call system as in claim 9, wherein said processing means is arranged for detecting false operation of said call button.

11. Nurse call system as in claim 9 or 10, wherein said processing means is arranged for detecting false operation of said status indicator.

## Patentansprüche

1. Schwesternrufanlage, umfassend
- eine zentrale Steuerung (10), die so eingerichtet ist, dass sie Schwesternrufmeldungen verarbeitet und verteilt,
- eine Eingabe-/Ausgabevorrichtung, die mit der zentralen Steuerung über eine erste Nachrichtenverbindung (11) eines Schwesternrufnetzwerkes verbunden ist,
- ein Handgerät zum Rufen einer Schwester, wobei das Handgerät so eingerichtet ist, dass es mit der Eingabe-/Ausgabevorrichtung über eine drahtgebundene elektrische Verbindung (5) kommuniziert, und eine Ruftaste (2), die mit einer Statusanzeige (1) versehen ist, die anzeigt, dass ein Ruf vorliegt oder nicht, und ein Verarbeitungsmittel (4) umfasst, mit dem die Statusanzeige und die Ruftaste überwacht werden können, wobei das Handgerät mit der zentralen Steuerung über die erste Nachrichtenverbindung durch die Eingabe-/Ausgabevorrichtung verbunden ist,
**dadurch gekennzeichnet, dass** das Verarbeitungsmittel des Handgerätes so eingerichtet ist, dass es bei Erfassung einer Fehlschaltung der Kommunikation zwischen dem Handgerät und der Eingabe-/Ausgabevorrichtung an der Hardwareebene als zweiten Pfad eine zweite Netzwerk-Nachrichtenverbindung (6) zwischen dem Handgerät und der zentralen Steuerung (10) aufbaut, wodurch die Eingabe-/Ausgabevorrichtung umgangen wird.

2. Schwesternrufanlage nach Anspruch 1, wobei die drahtgebundene elektrische Verbindung dem Standard DIN VDE 0834 entspricht.

3. Schwesternrufanlage nach Anspruch 1 oder 2, wobei das Handgerät eine Anzeige umfasst.

4. Schwesternrufanlage nach Anspruch 3, wobei die Anzeige so eingerichtet ist, dass sie einen Hinweis auf die Fehlschaltung anzeigt.

5. Schwesternrufanlage nach einem der vorherigen Ansprüche, wobei das Verarbeitungsmittel (4) so angepasst ist, dass es einen Netzwerkfehler in der zweiten Nachrichtenverbindung erfasst.

6. Schwesternrufanlage nach Anspruch 5, wobei das Handgerät so eingerichtet ist, dass es Feedback bezüglich der Erfassung des Netzwerkfehlers bereitstellt.

7. Schwesternrufanlage nach einem der vorherigen Ansprüche, wobei die Statusanzeige so eingerichtet ist, dass sie ein bestimmtes Lichtmuster ausgibt, um die Fehlschaltung zu signalisieren.

8. Schwesternrufanlage nach einem der vorherigen Ansprüche, wobei die zweite Nachrichtenverbindung eine Ethernet-Verbindung ist.

9. Schwesternrufanlage nach einem der vorherigen Ansprüche, wobei die Ruftaste (2) eine Taste mit Ruhekontakt ist.

10. Schwesternrufanlage nach Anspruch 9, wobei das Verarbeitungsmittel so eingerichtet ist, dass es ein Falschansprechen der Ruftaste erfasst.

11. Schwesternrufanlage nach Anspruch 9 oder 10, wobei das Verarbeitungsmittel so eingerichtet ist, dass es ein Falschansprechen der Statusanzeige erfasst.

## Revendications

1. Système d'appel infirmier comprenant
- un contrôleur central (10) agencé pour traiter et distribuer des messages d'appel infirmier,
- un dispositif d'entrée/de sortie connecté audit contrôleur central par l'intermédiaire d'une première liaison de communication (11) d'un réseau d'appel infirmier,
- un combiné pour effectuer un appel infirmier, ledit combiné étant agencé pour communiquer avec ledit dispositif d'entrée/de sortie par l'intermédiaire d'une connexion électrique câblée (5) et comprenant un bouton d'appel (2) doté d'un indicateur d'état (1) pour indiquer qu'un appel est présent ou non, et un moyen de traitement (4) capable de surveiller l'indicateur d'état et ledit bouton d'appel, ledit combiné étant connecté audit contrôleur central par l'intermédiaire de ladite première liaison de communication au moyen dudit dispositif d'entrée/de sortie,
**caractérisé en ce que** ledit moyen de traitement dudit combiné est agencé pour, lors de la détection au niveau matériel d'une anomalie de fonctionnement de la communication entre le combiné et le dispositif d'entrée/de sortie, établir en tant que seconde voie une seconde liaison de communication réseau (6) entre ledit combiné et ledit contrôleur central (10), moyennant quoi ledit dispositif d'entrée/de sortie est contourné.

2. Système d'appel infirmier selon la revendication 1, dans lequel ladite connexion électrique câblée est conforme à la norme DIN VDE 0834.

3. Système d'appel infirmier selon la revendication 1 ou 2, dans lequel ledit combiné comprend un écran.

4. Système d'appel infirmier selon la revendication 3, dans lequel ledit écran est agencé pour afficher une indication de ladite anomalie de fonctionnement.

5. Système d'appel infirmier selon l'une quelconque des revendications précédentes, dans lequel ledit moyen de traitement (4) est adapté pour détecter un incident réseau dans ladite seconde liaison de communication.

6. Système d'appel infirmier selon la revendication 5, dans lequel ledit combiné est agencé pour fournir une rétroaction lors de la détection dudit incident réseau.

7. Système d'appel infirmier selon l'une quelconque des revendications précédentes, dans lequel ledit indicateur d'état est agencé pour émettre un motif de lumière dédié pour signaler ladite anomalie de fonctionnement.

8. Système d'appel infirmier selon l'une quelconque des revendications précédentes, dans lequel ladite seconde liaison de communication est une connexion Ethernet.

9. Système d'appel infirmier selon l'une quelconque des revendications précédentes, dans lequel ledit bouton d'appel (2) est un bouton normalement fermé.

10. Système d'appel infirmier selon la revendication 9, dans lequel ledit moyen de traitement est agencé pour détecter un mauvais fonctionnement dudit bouton d'appel.

11. Système d'appel infirmier selon la revendication 9 ou 10, dans lequel ledit moyen de traitement est agencé pour détecter un mauvais fonctionnement dudit indicateur d'état.
